# EUROPÄISCHE PATENTSCHRIFT

(11) **EP 3 945 968 B1**
(45) Veröffentlichungstag und Bekanntmachung des Hinweises auf die Patenterteilung: **20.03.2024**
(21) Anmeldenummer: 20720739.0
(22) Anmeldetag: 30.03.2020
(51) Int. Cl.: A47J 47/00

(54) **SCHNEIDBRETT**
CHOPPING BOARD
PLANCHE À DÉCOUPER

(30) Priorität: 03.04.2019 DE 102019108711
(43) Veröffentlichungstag der Anmeldung: 09.02.2022
(73) Patentinhaber: Aurora Life Science GmbH, 64295 Darmstadt (DE)
(72) Erfinder: BORAS, Philo, 64287 Darmstadt (DE); OBERTHÜR, Jonathan, 64283 Darmstadt (DE)
(74) Vertreter: Habermann Intellectual Property Partnerschaft von Patentanwälten mbB
(86) Internationale Anmeldenummer: PCT/EP2020/058908
(87) Internationale Veröffentlichungsnummer: WO 2020/201192

(56) Entgegenhaltungen:
- EP-A1- 2 548 469
- EP-A1- 2 840 932
- WO-A1-2018/202375
- US-A1- 2018 263 425
- US-B1- 10 101 521

## Beschreibung

Die Erfindung betrifft ein Schneidbrett für Lebensmittel mit einem Unterteil und zumindest einem lösbar auf dem Unterteil anordnenbaren Oberteil, wobei auf einer, bei bestimmungsgemäßer Anordnung des Oberteils auf dem Unterteil, dem Unterteil abgewandten Oberteiloberseite eine Schneidauflagefläche ausgebildet ist, auf der Schneidgut anordnenbar und schneidbar ist, wobei das Oberteil auf einer der Schneidauflagefläche gegenüberliegenden und dem Unterteil zugewandten Oberteilunterseite Verbindungselemente aufweist, mittels derer das Oberteil auf dem Unterteil aufsteht, wobei das Unterteil des Schneidbretts auf einer dem Oberteil abgewandten Unterteilunterseite zumindest eine Auflagefläche aufweist, mit der das Schneidbrett auf einem Untergrund aufliegt.

Sowohl im privaten, als auch im professionellen Bereich sind Köchinnen und Köche heutzutage mit einer Vielzahl von Utensilien konfrontiert. Mit steigender Anzahl der zur Verfügung stehenden Kochutensilien steigen jedoch die Anforderungen an die Arbeits- und Arbeitsplatzorganisation in einer Küche. Ein Ansatz zur Lösung dieses Problems ist das in der EP 2840932 B1 beschriebene Schneidbrett. Das dort offenbarte Schneidbrett ist eine Küchenunterlage, die Halterungen für standardisierte Küchenbehälter aufweist, die es ermöglichen, dass um eine durch das Brett gebildete Arbeitsfläche herum, Aufnahmemöglichkeiten für ungeschnittene Zutaten, geschnittene Zutaten sowie für Abfall geschaffen werden. Hierdurch ist eine Arbeitsorganisation beim Verarbeiten von Schneidgut verbessert. Nachteilig ist bei dem offenbarten Schneidbrett jedoch, dass es nach wie vor erforderlich ist, die einzelnen Zutaten, bzw. zu verarbeitenden Lebensmittel, mittels einer separaten Waage abzuwiegen.

Ein weiterer Aspekt, der im Bereich der modernen Gastronomie aber auch beim Kochen zuhause in den letzten Jahren mehr und mehr an Bedeutung gewonnen hat, ist die Qualität der verarbeiteten Lebensmittel. Qualität meint in diesem Zusammenhang zum einen die Frische von Lebensmitteln, beispielsweise von Fisch, Fleisch, Pilzen oder ähnlichem, sowie den Reifegrad, beispielsweise von Früchten. Die Lebensmittelqualität kann insbesondere mittels optischer Messverfahren bestimmt werden. Nachteilig bei den bekannten Messverfahren ist, dass sie mittels separater Gerätschaften durchgeführt werden müssen, deren Verwendung die Arbeitsabläufe in einer Küche stört, weshalb häufig von deren Nutzung abgesehen wird. WO 2018/202375 A1 offenbart ein Schneidbrett, welches einen Körper mit einer Oberfläche zum Schneiden des Schneidguts aufweist. Diese Oberfläche ist aus Glas, sodass sie dazu geeignet ist, mit Photodioden Messungen durchzuführen. EP 2 548 469 A1 offenbart eine Haltevorrichtung zum Halten einer Waage in einer Öffnung einer Tischplatte, sowie eine Wiegevorrichtung, die eine Waage und die Haltevorrichtung aufweist.

Als Aufgabe der vorliegenden Erfindung wird es daher angesehen, ein Schneidbrett zur Verfügung zu stellen, mittels dessen eine Bestimmung von Parametern von zu verarbeitenden Lebensmitteln erleichtert ist.

Diese Aufgabe wird erfindungsgemäß durch die Merkmale des Anspruchs 1 gelöst.

Durch eine derartige Ausgestaltung des erfindungsgemäßen Schneidbretts wird es ermöglicht, dass das Schneidgut auf einfache Art und Weise während der Verarbeitung vor den Sensor gehalten, und so eine Bestimmung der Qualität des Schneidguts durchgeführt werden kann.

Optisches Element im Sinne des Erfindungsgedankens meint insbesondere Linsen, Glasscheiben, Lupen, Lichtwellenleiter, Kameraanordnungen oder ähnliches. Je nachdem welche Art elektromagnetischer Strahlung zur Untersuchung des Schneidguts verwendet wird, ist die Verwendung unterschiedlicher optischer Elemente vorgesehen. Darüber hinaus ist es auch möglich und erfindungsgemäß vorgesehen, dass mehrere optische Elemente mit einem oder mehreren Sensoren vorgesehen sein können.

Es ist auch möglich und vorgesehen, dass das optische Element einen Filter aufweisen kann, bzw. lediglich für ein bestimmtes Spektrum elektromagnetischer Strahlung transparent ist. So sind beispielsweise Kunststoffe bekannt, die lediglich für Infrarotstrahlung transparent sind. Darüber hinaus kann das optische Element auch einen Polarisationsfilter aufweisen.

Es ist vorgesehen, dass der Sensor insbesondere ein Interferometer, ein Fotosensor, ein Geigerzähler oder ähnliches sein kann. Die Verwendung eines Geigerzählers kann insbesondere sinnvoll sein, wenn Wildbret oder Pilze verarbeitet werden, die mitunter erhöht strahlenbelastet sind, insbesondere mit Cäsium 137. Für eine gute Detektion der eventuell vorhandenen Strahlenbelastung ist es hierbei erfindungsgemäß vorgesehen, dass das optische Element aus einem Material gefertigt ist, das einen Durchtritt von Betastrahlung ermöglicht.

Darüber hinaus ist es erfindungsgemäß auch vorgesehen, dass sowohl der Strahlungssensor, als auch das optische Element wechselbar, bzw. austauschbar ausgeführt sein können. Das optische Element kann insbesondere auch derart ausgeführt sein, dass es mit bekannten optischen Wechselsystemen, beispielsweise für Kameraobjektive, kompatibel ist.

Eine vorteilhafte Umsetzung des Erfindungsgedankens sieht vor, dass das Oberteil des Schneidbretts, mit Ausnahme des optischen Elements, aus einem Material hergestellt ist, das für elektromagnetische Strahlung, insbesondere für sichtbares Licht, undurchlässig ist. Durch eine derartige Ausgestaltung des erfindungsgemäßen Schneidbretts ist es möglich, dass das Oberteil entsprechend ästhetischer Bedürfnisse eines Nutzers ausgeführt sein kann. Das Oberteil kann beispielsweise aus Massivholz, aus Metall, aus Kunststoff oder Ähnlichem gefertigt sein.

Eine vorteilhafte Ausgestaltung der Erfindung sieht vor, dass dem Strahlungssensor eine elektromagnetische Strahlungsquelle, insbesondere eine Lichtquelle zugeordnet ist, wobei die Strahlungsquelle derart mit dem optischen Element in Wirkverbindung gebracht ist, dass das Schneidgut durch die Strahlungsquelle bestrahlbar, insbesondere beleuchtbar ist und wobei eine Strahlungsausbreitung der durch die Strahlungsquelle emittierten Strahlung so anpassbar ist, dass durch das Schneidgut reflektierte Strahlung mittels des optischen Elements dem Strahlungssensor zuleitbar ist. Durch eine derartige Ausgestaltung des erfindungsgemäßen Schneidbretts wird es ermöglicht, dass das Schneidgut mittels eines Verfahrens, das Ähnlichkeit zur Auflichtmikroskopie aufweist, untersucht werden kann.

Darüber hinaus ist es ebenfalls möglich und erfindungsgemäß vorgesehen, dass die emittierte Strahlung ein Frequenzspektrum aufweisen kann, das eine Fluoreszenz des Schneidguts anregt, sodass eine Untersuchung des Schneidguts mittels eines Verfahrens durchgeführt werden kann, das Analogien zur Fluoreszenzmikroskopie aufweist.

Bei einer vorteilhaften Ausgestaltung des erfindungsgemäßen Schneidbretts ist vorgesehen, dass das Unterteil Kraftsensoren aufweist, wobei das Oberteil mit den Verbindungselementen auf den Kraftsensoren aufsteht und wobei eine durch das Oberteil und ein auf dem Oberteil angeordnetes Schneidgut ausgeübte Gewichtskraft vollständig in die Kraftsensoren eingeleitet wird. Es ist erfindungsgemäß vorgesehen, dass das derart ausgestaltete Schneidbrett auch eine Wägefunktion aufweisen kann. Vorteilhaft ist hierbei insbesondere, dass auf eine separate Küchenwaage verzichtet werden kann und Arbeitsabläufe in der Küche vereinfacht werden können.

Besonders vorteilhaft ist bei dem erfindungsgemäßen Schneidbrett, dass das abnehmbare Oberteil unabhängig von dem die Messelektronik aufweisenden Unterteil reinigbar ist. So ist es insbesondere vorgesehen, dass das Oberteil derart ausgestaltet sein kann, dass es in einem Geschirrspüler, insbesondere auch einem Haushaltsgeschirrspüler abwaschbar ist. Hierdurch ist die Alltagstauglichkeit des Schneidbretts erhöht und die Hygiene in der Küche verbessert.

Eine vorteilhafte Umsetzung des Erfindungsgedankens sieht vor, dass das Unterteil zumindest eine Haltevorrichtung zur Aufnahme eines Küchenutensils aufweist. Küchenutensil im Sinne des Erfindungsgedankens meint insbesondere Messer, Rührgeräte, Schüsseln, Messbecher oder Ähnliches

Bei einer besonders vorteilhaften Umsetzung des Erfindungsgedankens ist vorgesehen, dass die Haltevorrichtung zur Aufnahme eines Behälters ausgebildet ist, wobei die Haltevorrichtung derart an den Behälter angepasst ist, dass der Behälter bei bestimmungsgemäßer Anordnung in der Haltevorrichtung zumindest abschnittsweise mit einem in seinem Öffnungsbereich auskragend ausgebildeten Rand auf der Haltevorrichtung aufliegt. Hierbei ist es vorgesehen, dass die Haltevorrichtung insbesondere an standardisierte Küchenbehälter, beispielsweise sogenannte "Gastronorm-Behälter" nach DIN 66075, angepasst sein kann. Dadurch, dass die Haltevorrichtung an dem Unterteil angeordnet ist, haben an der Haltevorrichtung angeordnete Gegenstände keine Einfluss auf die erfindungsgemäße Wägefunktion des Schneidbretts.

Eine vorteilhafte Ausgestaltung der Erfindung sieht vor, dass das Schneidbrett eine Datenverarbeitungseinrichtung aufweist, wobei der Strahlungssensor und/oder die Kraftsensoren signalleitend mit der Datenverarbeitungseinrichtung verbunden/verbindbar sind, wobei mittels der Datenverarbeitungseinrichtung durch die Sensoren erzeugte Signale auswertbar und Sensordaten erzeugbar sind. Durch eine derartige Integration der Datenverarbeitungseinrichtung in das erfindungsgemäße Schneidbrett wird erreicht, dass das Schneidbrett als ein sogenanntes "Smart-Device" ausgebildet sein kann.

Bei einer besonders vorteilhaften Umsetzung des Erfindungsgedankens ist vorgesehen, dass die Datenverarbeitungseinrichtung in dem Unterteil angeordnet ist. Hierdurch wird erreicht, dass das Oberteil, das mit dem Schneidgut in Kontakt kommt und gegebenenfalls verunreinigt ist, unabhängig von dem Unterteil, das bei bestimmungsgemäßem Gebrauch des Schneidbretts keinen Kontakt zu dem Schneidgut hat, gereinigt werden kann. Dies ist insbesondere vorteilhaft, da die Datenverarbeitungseinrichtung nicht derart ausgelegt werden muss, dass sie den bei einem Reinigungsvorgang auftretenden Umwelteinflüssen widersteht. Dies wirkt sich insbesondere positiv auf die zu erwartenden Produktionskosten des erfindungsgemäßen Schneidbretts aus, da eine Härtung von elektronischen Komponenten insbesondere gegen Wasser und Wasserdampf kostenintensiv und aufwendig ist.

Bei einer vorteilhaften Ausgestaltung des erfindungsgemäßen Schneidbretts ist vorgesehen, dass in der Datenverarbeitungseinrichtung eine Datenauswerteregel hinterlegt ist, auf Grundlage derer aus den Sensordaten Auswertedaten erzeugbar sind. Durch eine derartige Ausgestaltung des erfindungsgemäßen Schneidbretts ist es ermöglicht, dass beispielsweise bei verderblichen Lebensmitteln aus deren Infrarotstrahlung ihre Temperatur bestimmt und anhand der Auswerteregel festgelegt werden kann, ob diese noch für die Weiterverarbeitung geeignet sind oder nicht, wobei die Auswertedaten in diesem Fall beispielsweise die Werte "Ja", "Nein" oder "Nicht für den Rohverzehr geeignet!" umfassen können.

Eine vorteilhafte Umsetzung des Erfindungsgedankens sieht vor, dass die Datenverarbeitungseinrichtung einen Datenspeicher aufweist und/oder mit einem Datenspeicher in Wirkverbindung bringbar ist, in dem die Sensordaten und/oder Auswertedaten speicherbar sind. Hierdurch ist es beispielsweise möglich, dass eine Verarbeitungstemperatur von Lebensmitteln über einen gewissen Zeitraum protokolliert werden kann.

Es ist hierbei auch möglich und vorgesehen, dass der Datenspeicher, insbesondere plombiert, ausgeführt sein kann, sodass eine Manipulation der gespeicherten Daten verhindert wird, um so eine Qualitätskontrolle im Sinne der Lebensmittelhygiene nachweisen zu können. Bei einer besonders vorteilhaften Ausgestaltung der Erfindung ist vorgesehen, dass der Datenspeicher als ein Wechseldatenspeicher ausgeführt sein kann, beispielsweise eine Speicherkarte, insbesondere eine SD-Karte, oder ein USB-Stick.

Eine vorteilhafte Ausgestaltung der Erfindung sieht vor, dass durch die Datenverarbeitungseinrichtung aus den in den Datenspeicher hinterlegten Sensordaten und/oder Auswertedaten Sensordatensätze und/oder Auswertedatensätze bildbar sind, wobei die gebildeten Datensätze in dem Datenspeicher speicherbar sind. Hierbei ist es insbesondere möglich und erfindungsgemäß vorgesehen, dass in den gebildeten Auswertedatensätzen die Sensordaten und/oder Auswertedaten mit weiteren Daten verknüpft sein können, insbesondere einem Zeitstempel, einer Nutzer-ID oder ähnlichem.

Bei einer vorteilhaften Ausgestaltung des erfindungsgemäßen Schneidbretts ist vorgesehen, dass das Schneidbrett eine Anzeigevorrichtung aufweist, wobei die Anzeigevorrichtung mit der Datenverarbeitungseinrichtung in Wirkverbindung bringbar/gebracht ist und wobei Sensordaten und/oder Auswertedaten und/oder Sensordatensätze und/oder Auswertedatensätze mittels der Anzeigevorrichtung visualisierbar sind. Hierbei ist es insbesondere vorgesehen, dass mittels der Anzeigevorrichtung Temperaturen, Lebensmittelbezeichnungen, Temperaturverläufe, Lebensmittelqualitäten, Verarbeitungshinweise oder ähnliches angezeigt werden können.

Bei einer besonders vorteilhaften Ausgestaltung des erfindungsgemäßen Schneidbretts ist vorgesehen, dass die Anzeigevorrichtung mit einer berührungsempfindlichen Eingabevorrichtung verbunden ist, sodass eine Interaktion zwischen einem Nutzer des Schneidbretts und dem Schneidbrett ermöglicht ist.

Eine vorteilhafte Umsetzung des Erfindungsgedankens sieht vor, dass das Schneidbrett eine Datenübertragungsvorrichtung aufweist, wobei mittels der Datenübertragungsvorrichtung Sensordaten und/oder Auswertedaten und/oder Sensordatensätze und/oder Auswertedatensätze an ein externes Gerät, insbesondere ein Smartphone übertragbar sind. Bei einer derartigen Ausgestaltung des erfindungsgemäßen Schneidbretts wird es ermöglicht, dass die mittels der Datenübertragungsvorrichtung übertragenen Daten durch das externe Gerät ausgewertet werden können.

Bei einer besonders vorteilhaften Ausgestaltung der Erfindung ist es vorgesehen, dass auf dem externen Gerät eine spezialisierte Software ausgeführt wird, die an das erfindungsgemäße Schneidbrett angepasst ist und die eine Auswertung, Anzeige, Speicherung oder ähnliches der durch das Schneidbrett ermittelten Daten ermöglicht. Dabei ist es auch möglich und erfindungsgemäß vorgesehen, dass die Übertragung der Daten nicht ausschließlich mittels der Datenübertragungsvorrichtung erfolgen muss, sondern dass es beispielsweise auch möglich ist, dass der Datenspeicher des erfindungsgemäßen Schneidbretts in das externe Gerät eingesetzt wird und die Daten so übertragen werden können.

Bei einer weiteren besonders vorteilhaften Ausführungsform der Erfindung ist vorgesehen, dass auf der Grundlage der mittels des erfindungsgemäßen Schneidbretts ermittelten Daten beispielsweise Ernährungspläne erstellt, Gesundheitshinweise ermittelt, Ernährungsverhalten protokolliert oder ähnliches durchgeführt wird.

Nachfolgend werden einige Ausführungsbeispiele des Erfindungsgedankens näher erläutert, in den Zeichnungen dargestellt sind. Es zeigen:
Figuren 1, 2 und 4 schematische Darstellung von Ausführungsformen des Schneidbretts und
Figur 3 eine vereinfachte schematische Darstellung der Verbindungen zwischen elektronischen Komponenten des Schneidbretts.

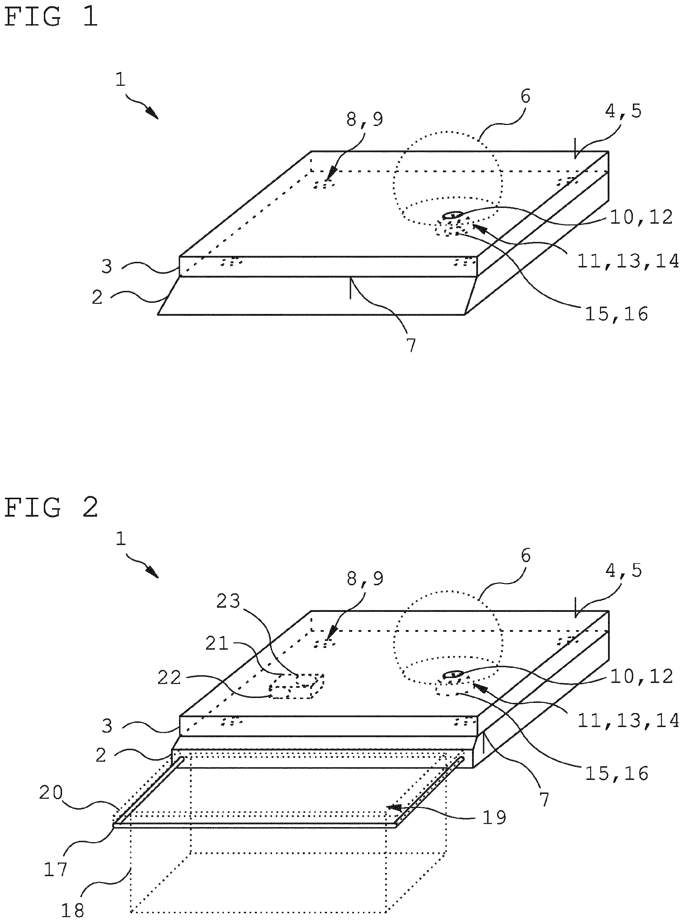

In Figur 1 ist eine schematische Darstellung eines erfindungsgemäßen Schneidbretts 1 für Lebensmittel gezeigt. Das dargestellte Schneidbrett 1 weist ein Unterteil 2 und ein Oberteil 3 auf. Das Oberteil 3 ist auf das Unterteil 2 aufgelegt und von diesem lösbar. Auf einer Oberteiloberseite 4 ist eine Schneidauflagefläche 5 ausgebildet. Bei der dargestellten Ausführungsform des erfindungsgemäßen Schneidbretts 1 ist die komplette Oberteiloberseite 4 als Schneidauflagefläche 5 ausgebildet. Auf der Schneidauflagefläche 5 ist Schneidgut 6 anordnenbar und schneidbar.

Das Oberteil 3 weist auf einer der Schneidauflagefläche gegenüberliegenden und dem Unterteil zugewandten Oberteilunterseite 7 Verbindungselemente 8 auf. Mittels dieser Verbindungselemente 8 steht das Oberteil 3 auf dem Unterteil 2 auf. Bei der dargestellten Ausführungsform eines erfindungsgemäßen Schneidbretts 1 stehen die Verbindungselemente 8 in auf dem Unterteil 2 ausgebildeten Ausnehmungen (nicht bezeichnet) auf Kraftsensoren 9 auf. Eine durch das Oberteil 3 und das Schneidgut 6 ausgeübte Gewichtskraft wird vollständig in die Kraftsensoren 9 eingeleitet.

Das Oberteil 3 der dargestellten Ausführungsform des erfindungsgemäßen Schneidbretts 1 weist ein optisches Element 10 auf. Das optische Element 10 umfasst ein das Oberteil 3 vollständig durchdringende, kreisrund Sensorausnehmung 11 sowie eine in der Sensorausnehmung 11 angeordnete und bündig mit der Schneidauflagefläche 5 abschließende transparente Scheibe 12. Mittels der Scheibe 12 und der Sensorausnehmung 11 ist eine Ausbreitung von durch das Schneidgut 6 emittiertem sichtbarem Licht durch das Oberteil 3 hindurch ermöglicht.

In einem dem optischen Element 10 zugeordneten Sensorbereich 13 des Unterteils 2 ist in einer an die Sensorausnehmung 11 angepassten Sensoraufnahme 14 ein Interferometer 15 angeordnet, mittels dessen die elektromagnetische Strahlung detektierbar ist, sodass Eigenschaften des Schneidguts 6 bestimmbar sind.

Das Schneidbrett 1 weist auch eine Lichtquelle 16 auf, die benachbart zu dem Interferometer 15 in der Sensoraufnahme 14 angeordnet ist. Mittels der Lichtquelle 16 ist das Schneidgut 6 durch das optische Element 10 hindurch mit sichtbarem Licht bestrahlbar.

Anders als bei der in Figur 1 dargestellt Ausführungsform, weist bei der in Figur 2 dargestellte Ausführungsform des erfindungsgemäßen Schneidbretts 1 das Unterteil 2 eine Haltevorrichtung 17 zur Aufnahme eines Behälters 18 auf, wobei ein exemplarischer Behälter 18 in der Figur 2 gezeigt ist. Die Haltervorrichtung 17 ist derart an den Behälter 18 angepasst, dass der Behälter 18 abschnittsweise mit einem in seinem Öffnungsbereich 19 auskragend ausgebildeten Rand 20 auf der Haltevorrichtung 17 aufliegt.

Die dargestellte Ausführungsform des erfindungsgemäßen Schneidbretts 1 weist auch eine Datenverarbeitungseinrichtung 21 auf, die mit den Kraftsensoren 9 und dem Interferometer 15 in Wirkverbindung gebracht ist. Darüber hinaus weist die Datenverarbeitungseinrichtung 21 einen Datenspeicher 22 und eine Datenübertragungsvorrichtung 23 auf.

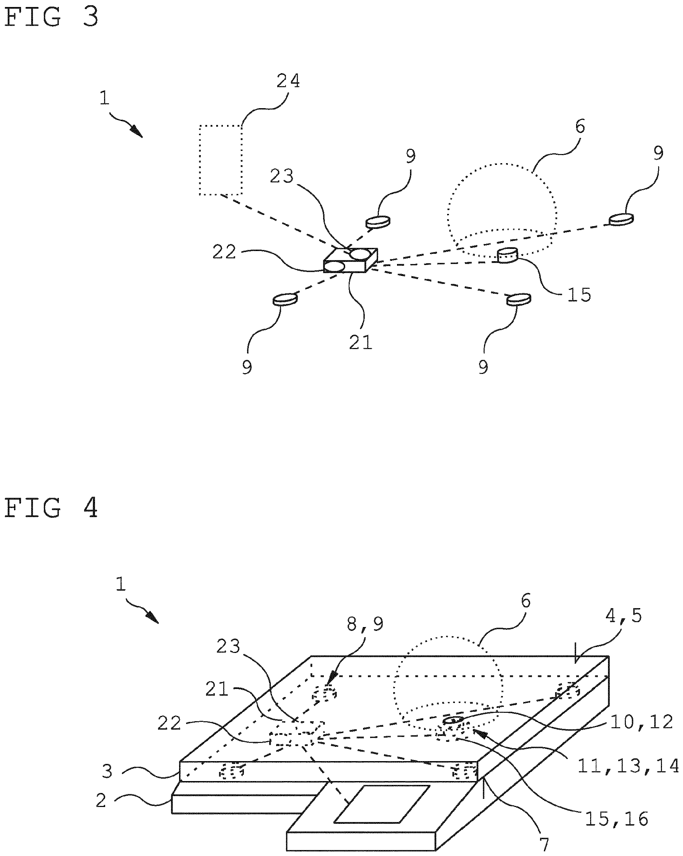

In Figur 3 ist schematisch dargestellt, wie bei der in Figur 2 dargestellten Ausführungsform des Schneidbretts 1 die Datenverarbeitungseinrichtung 21 signalleitend mit dem Interferometer 15 und den Kraftsensoren 9 verbunden ist. Mittels der Datenverarbeitungseinrichtung 21 können die durch die Sensoren 9, 15 erzeugte Signale ausgewertet und Sensordaten erzeugt werden. 7. Schneidbrett nach Anspruch In der Datenverarbeitungseinrichtung 21 ist auch eine Auswerteregel hinterlegt, auf Grundlage derer aus den Sensordaten Auswertedaten erzeugt werden können. Die Sensordaten und die Auswertedaten können in dem Datenspeicher 22 gespeichert werden.

In Figur 3 ist auch ein externes Gerät 24 gezeigt an das von der Datenverarbeitungseinrichtung 21 mittels der Datenübertragungsvorrichtung 23 Daten übertragen werden. Aus den Daten sind mittels des externen Gerätes 24 Informationen über das Schneidgut ermittelbar.

Die in Figur 4 dargestellte Ausführungsform des erfindungsgemäßen Schneidbretts 1 weist eine integrierte Anzeigevorrichtung 25 auf, die mit der Datenverarbeitungseinrichtung 21 signalleitend verbunden ist. Mittels der Anzeigevorrichtung 25 sind die durch die Datenverarbeitungseinrichtung 21 ermittelten Auswertedaten visualisierbar.

### BEZUGSZEICHENLISTE

- 1.: Schneidbrett
- 2.: Unterteil
- 3.: Oberteil
- 4.: Oberteiloberseite
- 5.: Schneidauflagefläche
- 6.: Schneidgut
- 7.: Oberteilunterseite
- 8.: Verbindungselement
- 9.: Kraftsensor
- 10.: optisches Element
- 11.: Sensorausnehmung
- 12.: Scheibe
- 13.: Sensorbereich
- 14.: Sensoraufnahme
- 15.: Interferometer
- 16.: Lichtquelle
- 17.: Haltevorrichtungen
- 18.: Behälter
- 19.: Öffnungsbereich
- 20.: Rand
- 21.: Datenverarbeitungseinrichtung
- 22.: Datenspeicher
- 23.: Datenübertragungsvorrichtung
- 24.: externes Gerät
- 25.: Anzeigevorrichtung

## Patentansprüche

1. Schneidbrett (1) für Lebensmittel mit einem Unterteil (2) und zumindest einem lösbar auf dem Unterteil (2) anordnenbaren Oberteil (3), wobei auf einer, bei bestimmungsgemäßer Anordnung des Oberteils (3) auf dem Unterteil (2), dem Unterteil (2) abgewandten Oberteiloberseite (4) eine Schneidauflagefläche (5) ausgebildet ist, auf der Schneidgut (6) anordnenbar und schneidbar ist, wobei das Oberteil (3) auf einer der Schneidauflagefläche (5) gegenüberliegenden und dem Unterteil (2) zugewandten Oberteilunterseite (7) Verbindungselemente (8) aufweist, mittels derer das Oberteil (3) auf dem Unterteil (2) aufsteht und wobei das Unterteil (2) des Schneidbretts (1) auf einer dem Oberteil (3) abgewandten Unterteilunterseite zumindest eine Auflagefläche aufweist, mit der das Schneidbrett (1) auf einem Untergrund aufliegt, wobei das Oberteil (3) zumindest ein optisches Element (10) aufweist, mittels dessen eine Ausbreitung von durch das Schnittgut emittierter und/ oder reflektierter elektromagnetischer Strahlung, insbesondere von elektromagnetischer Strahlung im Nahinfrarotbereich, durch das Oberteil (3) hindurch ermöglicht ist, wobei in einem dem optischen Element (10) zugeordneten Sensorbereich des Unterteils (2) zumindest ein Strahlungssensor zum Erfassen elektromagnetischer Strahlung, insbesondere ein Interferometer (15), angeordnet ist, mittels dessen die elektromagnetische Strahlung detektierbar ist, sodass Eigenschaften des Schneidguts (6) bestimmbar sind.

2. Schneidbrett (1) nach Anspruch 1, **dadurch gekennzeichnet, dass** das Oberteil (3) des Schneidbretts (1), mit Ausnahme des optischen Elements (10), aus einem Material hergestellt ist, dass für elektromagnetische Strahlung, insbesondere für sichtbares Licht, undurchlässig ist.

3. Schneidbrett (1) nach einem der Ansprüche 1 oder 2, **dadurch gekennzeichnet, dass** dem Strahlungssensor eine elektromagnetische Strahlungsquelle, insbesondere eine Lichtquelle (16), zugeordnet ist, wobei die Strahlungsquelle derart mit dem optischen Element (10) in Wirkverbindung gebracht/ bringbar ist, dass das Schneidgut (6) durch die Strahlungsquelle bestrahlbar, insbesondere beleuchtbar, ist und wobei eine Strahlungsausbreitung der durch die Strahlungsquelle emittierten Strahlung so angepasst, anpassbar ist, dass durch das Schnittgut reflektierte Strahlung mittels des optischen Elements (10) dem Strahlungssensor zuleitbar ist.

4. Schneidbrett (1) nach einem der Anspruch 1 bis 3, **dadurch gekennzeichnet, dass** das Unterteil (2) Kraftsensoren (9) aufweist, wobei das Oberteil (3) mit den Verbindungselementen (8) auf den Kraftsensoren (9) aufsteht und wobei eine durch das Oberteil (3) und ein auf dem Oberteil (3) angeordnetes Schneidgut (6) ausgeübte Gewichtskraft vollständig in die Kraftsensoren (9) eingeleitet wird.

5. Schneidbrett (1) nach einem der Ansprüche 1 bis 4, **dadurch gekennzeichnet, dass** das Unterteil (2) zumindest eine Haltevorrichtung (17) zur Aufnahme eines Küchenutensils aufweist.

6. Schneidbrett (1) nach Anspruch 5, **dadurch gekennzeichnet, dass** das Unterteil (2) zumindest eine Haltevorrichtung (17) zur Aufnahme eines Behälters (18) aufweist, wobei die Haltervorrichtung derart an den Behälter angepasst ist, dass der Behälter bei bestimmungsgemäßer Anordnung in der Haltevorrichtung (17) zumindest abschnittsweise mit einem in seinem Öffnungsbereich (19) auskragend ausgebildeten Rand (20) auf der Haltevorrichtung (17) aufliegt.

7. Schneidbrett (1) nach einem der Ansprüche 1 bis 6, **dadurch gekennzeichnet, dass** Schneidbrett (1) eine Datenverarbeitungseinrichtung (21) aufweist, wobei der Strahlungssensor und/ oder die Kraftsensoren (9) signalleitend mit der Datenverarbeitungseinrichtung (21) verbunden/ verbindbar sind, wobei mittels der Datenverarbeitungseinrichtung (21) durch die Sensoren erzeugte Signale auswertbar und Sensordaten erzeugbar sind.

8. Schneidbrett (1) nach Anspruch 7, **dadurch gekennzeichnet, dass** in der Datenverarbeitungseinrichtung (21) eine Auswerteregel hinterlegt ist, auf Grundlage derer aus den Sensordaten Auswertedaten erzeugbar sind.

9. Schneidbrett (1) nach einem der Ansprüche 7 oder 8, **dadurch gekennzeichnet, dass** die Datenverarbeitungseinrichtung (21) einen Datenspeicher (22) aufweist und/ oder mit einem Datenspeicher (22) in Wirkverbindung bringbar ist, in dem die Sensordaten und/ oder Auswertedaten speicherbar sind.

10. Schneidbrett (1) nach Anspruch 9, **dadurch gekennzeichnet, dass** durch die Datenverarbeitungseinrichtung (21) aus den in dem Datenspeicher (22) hinterlegten Sensordaten und/ oder Auswertedaten Sensordatensätze und/ oder Auswertedatensätze bildbar sind, wobei die gebildeten Datensätze in dem Datenspeicher (22) speicherbar sind.

11. Schneidbrett (1) nach einem der Ansprüche 7 bis 10, **dadurch gekennzeichnet, dass** das Schneidbrett (1) eine Anzeigevorrichtung (25) aufweist, wobei die Anzeigevorrichtung (25) mit der Datenverarbeitungseinrichtung (21) in Wirkverbindung bringbar/ gebracht ist und wobei Sensordaten und/ oder Auswertedaten und/ oder Sensordatensätze und/ oder Auswertedatensätze mittels der Anzeigevorrichtung (25) visualisierbar sind.

12. Schneidbrett (1) nach einem der Ansprüche 7 bis 11, **dadurch gekennzeichnet, dass** das Schneidbrett (1) eine Datenübertragungsvorrichtung (23) aufweist, wobei mittels der Datenübertragungsvorrichtung (23) Sensordaten und/ oder Auswertedaten und/ oder Sensordatensätze und/ oder Auswertedatensätze an ein externes Gerät (24), insbesondere ein Smartphone, übertragbar sind.

## Claims

1. Chopping board (1) for foodstuffs, comprising a lower part (2) and at least one upper part (3) which can be detachably arranged on the lower part (2), wherein a chopping support surface (5) is formed on the upper part top side (4) facing away from the lower part (2), when the upper part (3) is arranged as intended on the lower part (2), on which chopping support surface material to be chopped (6) can be arranged and chopped, wherein the upper part (3) comprises connection elements (8) on an upper part underside (7) which is opposite the chopping support surface (5) and faces the lower part (2), by means of which connection elements the upper part (3) stands on the lower part (2), and wherein the lower part (2) of the chopping board (1) comprises at least one contact surface on a lower part underside facing away from the upper part (3), by means of which contact surface the chopping board (1) rests on a substrate, wherein the upper part (3) comprises at least one optical element (10), by means of which a propagation of electromagnetic radiation that is emitted and/or reflected by the material to be chopped, through the upper part (3), is made possible, wherein at least one radiation sensor for detecting electromagnetic radiation, in particular an interferometer (15), is arranged in a sensor region of the lower part (2) associated with the optical element (10), by means of which sensor the electromagnetic radiation can be detected, such that properties of the material to be chopped (6) can be determined.

2. Chopping board (1) according to claim 1, **characterised in that** the upper part (3) of the chopping board (1), with the exception of the optical element (10), is produced from a material which is impermeable to electromagnetic radiation, in particular for visible light.

3. Chopping board (1) according to either claim 1 or claim 2, **characterised in that** the radiation sensor is associated with an electromagnetic radiation source, in particular a light source (16), wherein the radiation source is or can be brought into operative connection with the optical element (10) in such a way that the material to be hopped (6) can be irradiated, in particular illuminated, by the radiation source, and wherein a radiation propagation of the radiation emitted by the radiation source is/can be adjusted in such a way that radiation reflected by the material to be chopped can be forwarded to the radiation sensor by means of the optical element (10).

4. Chopping board (1) according to any of claims 1 to 3, **characterised in that** the lower part (2) comprises force sensors (9), wherein the upper part stands on the force sensors (9) by means of the connection elements (8), and wherein a weight force exerted by the upper part (3) and material to be chopped (6) that is arranged on the upper part (3) is introduced fully into the force sensors (9).

5. Chopping board (1) according to any of claims 1 to 4, **characterised in that** the lower part (2) comprises at least one holding device (17) for receiving a kitchen utensil.

6. Chopping board (1) according to claim 5, **characterised in that** the lower part (2) comprises at least one holding device (17) for receiving a container (18), wherein the holding device is adapted to the container in such a way that, when arranged as intended in the holding device (17), the container rests on the holding device (17) at least in portions with an edge (20) formed in a cantilevered manner in its opening region (19).

7. Chopping board (1) according to any of claims 1 to 6, **characterised in that** the chopping board (1) comprises a data processing means (21), wherein the radiation sensor and/or the force sensors (9) are or can be connected to the data processing means (21) for signal transmission, wherein signals generated by the sensors can be evaluated and sensor data generated by means of the data processing means (21).

8. Chopping board (1) according to claim 7, **characterised in that** an evaluation rule is stored in the data processing means (21), on the basis of which rule evaluation data can be generated from the sensor data.

9. Chopping board (1) according to either claim 7 or claim 8, **characterised in that** the data processing means (21) comprises a data memory (22) and/or can be brought into operative connection with a data memory (22), in which the sensor data and/or evaluation data can be stored.

10. Chopping board (1) according to claim 9, **characterised in that** sensor datasets and/or evaluation datasets can be formed, by the data processing means (21), from the sensor data and/or evaluation data stored in the data memory (22), wherein the formed datasets can be stored in the data memory (22).

11. Chopping board (1) according to any of claims 7 to 10, **characterised in that** the chopping board (1) comprises a display device (25), wherein the display device (25) can be/is brought into operative connection with the data processing means (21), and wherein sensor data and/or evaluation data and/or sensor datasets and/or evaluation datasets can be visualised by means of the display device (25).

12. Chopping board (1) according to any of claims 7 to 11, **characterised in that** the chopping board (1) comprises a data transmission device (23), wherein sensor data and/or evaluation data and/or sensor datasets and/or evaluation datasets can be transmitted to an external device (24), in particular a smartphone, by means of the data transmission device (23).

## Revendications

1. Planche à découper (1) pour produits alimentaires avec une partie inférieure (2) et au moins une partie supérieure (3) pouvant être agencée de manière amovible sur la partie inférieure (2), une surface d'appui de coupe (5) étant réalisée sur un côté supérieur de partie supérieure (4) détourné de la partie inférieure (2) lorsque la partie supérieure (3) est agencée de manière conforme sur la partie inférieure (2), surface sur laquelle un produit à découper (6) peut être agencé et découpé, la partie supérieure (3) présentant, sur un côté inférieur de partie supérieure (7) opposé à la surface d'appui de coupe (5) et tourné vers la partie inférieure (2), des éléments de liaison (8) au moyen desquels la partie supérieure (3) se dresse sur la partie inférieure (2), et la partie inférieure (2) de la planche à découper (1) présentant, sur un côté inférieur de partie inférieure détourné de la partie supérieure (3), au moins une surface d'appui avec laquelle la planche à découper (1) repose sur un support, la partie supérieure (3) présentant au moins un élément optique (10) au moyen duquel une propagation d'un rayonnement électromagnétique émis et/ou réfléchi par le produit à découper, notamment d'un rayonnement électromagnétique dans la plage du proche infrarouge, est possible à travers la partie supérieure (3), dans une zone de capteur de la partie inférieure (2) associée à l'élément optique (10) étant agencé au moins un capteur de rayonnement pour détecter un rayonnement électromagnétique, notamment un interféromètre (15), au moyen duquel le rayonnement électromagnétique peut être détecté, de telle sorte que des propriétés du produit à découper (6) peuvent être déterminées.

2. Planche à découper (1) selon la revendication 1, **caractérisée en ce que** la partie supérieure (3) de la planche à découper (1), à l'exception de l'élément optique (10), est fabriquée en un matériau qui est opaque au rayonnement électromagnétique, notamment à la lumière visible.

3. Planche à découper (1) selon l'une quelconque des revendications 1 ou 2, **caractérisée en ce qu'**une source de rayonnement électromagnétique, notamment une source de lumière (16), est associée au capteur de rayonnement, la source de rayonnement étant amenée/pouvant être amenée en liaison active avec l'élément optique (10) de telle sorte que le produit à découper (6) peut être exposé à la source de rayonnement, notamment éclairé par celle-ci, et une propagation de rayonnement du rayonnement émis par la source de rayonnement étant adaptée, adaptable, de telle sorte que le rayonnement réfléchi par le produit à découper peut être transmis au capteur de rayonnement au moyen de l'élément optique (10).

4. Planche à découper (1) selon l'une quelconque des revendications 1 à 3, **caractérisée en ce que** la partie inférieure (2) présente des capteurs de force (9), la partie supérieure (3) se dressant avec les éléments de liaison (8) sur les capteurs de force (9), et une force de poids exercée par la partie supérieure (3) et un produit à découper (6) agencé sur la partie supérieure (3) étant entièrement introduite dans les capteurs de force (9).

5. Planche à découper (1) selon l'une quelconque des revendications 1 à 4, **caractérisée en ce que** la partie inférieure (2) présente au moins un dispositif de maintien (17) pour recevoir un ustensile de cuisine.

6. Planche à découper (1) selon la revendication 5, **caractérisée en ce que** la partie inférieure (2) présente au moins un dispositif de maintien (17) pour recevoir un récipient (18), le dispositif de maintien étant adapté au récipient de telle sorte que le récipient, lorsqu'il est agencé de manière conforme dans le dispositif de maintien (17), repose au moins par sections sur le dispositif de maintien (17) avec un bord (20) réalisé en saillie dans sa zone d'ouverture (19).

7. Planche à découper (1) selon l'une quelconque des revendications 1 à 6, **caractérisée en ce que** la planche à découper (1) présente un appareil de traitement de données (21), le capteur de rayonnement et/ou les capteurs de force (9) étant reliés/pouvant être reliés à l'appareil de traitement de données (21) de manière à conduire les signaux, des signaux générés par les capteurs pouvant être évalués et des données de capteurs pouvant être générées au moyen de l'appareil de traitement de données (21).

8. Planche à découper (1) selon la revendication 7, **caractérisée en ce qu'**une règle d'évaluation est enregistrée dans l'appareil de traitement de données (21), sur la base de laquelle des données d'évaluation peuvent être générées à partir des données de capteurs.

9. Planche à découper (1) selon l'une quelconque des revendications 7 ou 8, **caractérisée en ce que** l'appareil de traitement de données (21) présente une mémoire de données (22) et/ou peut être mis en liaison active avec une mémoire de données (22) dans laquelle les données de capteurs et/ou les données d'évaluation peuvent être stockées.

10. Planche à découper (1) selon la revendication 9, **caractérisée en ce que** l'appareil de traitement de données (21) permet de former des ensembles de données de capteurs et/ou des ensembles de données d'évaluation à partir des données de capteurs et/ou des données d'évaluation enregistrées dans la mémoire de données (22), les ensembles de données formés pouvant être stockés dans la mémoire de données (22) .

11. Planche à découper (1) selon l'une quelconque des revendications 7 à 10, **caractérisée en ce que** la planche à découper (1) présente un dispositif d'affichage (25), le dispositif d'affichage (25) pouvant être mis/étant mis en liaison active avec l'appareil de traitement de données (21), et des données de capteurs et/ou des données d'évaluation et/ou des ensembles de données de capteurs et/ou des ensembles de données d'évaluation pouvant être visualisés au moyen du dispositif d'affichage (25).

12. Planche à découper (1) selon l'une quelconque des revendications 7 à 11, **caractérisée en ce que** la planche à découper (1) présente un dispositif de transfert de données (23), des données de capteurs et/ou des données d'évaluation et/ou des ensembles de données de capteurs et/ou des ensembles de données d'évaluation pouvant être transférés à un appareil externe (24), notamment un smartphone, au moyen du dispositif de transfert de données (23).
